# EUROPEAN PATENT APPLICATION

(11) **EP 3 722 408 A1**
(43) Date of publication of application: **14.10.2020**
(21) Application number: 19763895.0
(22) Date of filing: 08.03.2019
(51) Int. Cl.: C12M 1/00, C12M 1/12

(54) **UPSTREAM PHASED-RETENTION PRODUCTION METHOD FOR BIOMACROMOLECULES, PRODUCTION MODULE, AND USE IN PRODUCTION**

(30) Priority: 09.03.2018 CN 201810194834
(71) Applicant: Genor Biopharma Co., Ltd, Shanghai 201203 (CN)
(72) Inventor: ZHOU, Joe, Shanghai 201203 (CN); LI, Zicai, Shanghai 201203 (CN); XU, Shuiqing, Shanghai 201203 (CN); KAN, Steven, Z., Shanghai 201203 (CN); LIN, Jun, Shanghai 201203 (CN); WANG, Jianqing, Shanghai 201203 (CN); DUAN, Qingtang, Shanghai 201203 (CN); AN, Mingyan, Shanghai 201203 (CN); LI, Minzhu, Shanghai 201203 (CN)
(74) Representative: Vitina, Maruta
(86) International application number: PCT/CN2019/077502
(87) International publication number: WO 2019/170145

(57) **Abstract**

The present disclosure provides a staged-retention method for upstream production of biomacromolecules, a production module used therein, and an application of the method/module in the industrial production of polypeptides, fusion proteins and immunoglobulin molecules. The method of the present disclosure can effectively improve the upstream production efficiency, yield and purity in the large-scale production of a recombinant protein.

## Description

### TECHNICAL FIELD

The present disclosure relates to the production of biological products, particularly to the industrial production of biomacromolecules, and more particularly to the upstream process of the preparation of polypeptides, fusion proteins and immunoglobulins.

### BACKGROUND

Biomacromolecules, especially fusion proteins and immunoglobulin molecules, have received extensive attention in the current biopharmaceutical field. Generally, the biomacromolecules are prepared through the following steps: upstream cell culture; downstream collecting, purifying, refining and concentrating of antibody molecules from cell culture fluid; and adding of the antibody molecules into a preparation solution suitable for humans.

The expression system used in the upstream preparation process of recombinant proteins is established mainly from the mammalian cells. Specifically, cells in a single storage tube of the working cell bank are activated, and then cultured sequentially in an erlenmeyer flask, 2-liter, 10-liter and 50-liter culture tanks and a 500-liter cell reactor for production. Since there are as many as 70-80 kinds of components in the mediums involved in the cell culture process, and various impurities, such as secreted proteases, small cell debris, host cell proteins, DNA and RNA molecules, may be accumulated in the cell culture process, the separation and purification will become complicated. At the same time, there are also incomplete antibody molecules or antibody molecules with different charge distribution due to redox in the cell culture system, so that it is required to perform protein A-sepharose affinity chromatography to collect the desired antibodies after the cells are removed by centrifugation and deep-bed filtration in the downstream process, which has a yield of greater than 95%. However, in addition to most of intact antibody molecules with Fc fragment, there are also some incomplete antibody molecules and impurities relevant to the antibody in property, resulting in great difficulty in the subsequent purification and refinement steps. Given the above, in order to ensure the high quality of the product or production repeatability, the yield will generally suffer from a certain loss in the downstream purification and refining steps.

In the existing large-scale industrial production of biomacromolecules, the upstream cell culture is often performed by perfusion culture and fed-batch culture, and also adopts different cell retention processes to achieve high-density cell culture while achieving the removal of impurities in the cell culture medium. Alternating Tangential Flow Filtration (ATF) technique is a state-of-the-art retention process, which guarantees low shear force, large filtration area and strong anti-blockage capacity to cells, facilitating the high-density cell filtration. However, in the actual operation, due to the continuous growth of cells, metabolic products or product-related impurities with different properties in addition to the expressed protein will be accumulated during the large-scale culture of cells, for example, the cells mainly generate metabolites in the medium in the early stage of culture, at the same time, a small number of antibody products will be produced in the culture medium during the early stage of the rapid proliferation of cells, which are susceptible to the high culture temperature to undergo changes such as deamination or degradation to form product-related substances or impurities. The concentration of these metabolites and product-related impurities increases with the increase of concentration of cells and the desired protein, which will directly affect the yield and purity of the final protein product and the natural conformation of the protein product, so that the proteins will aggregate when accumulating in high concentration, resulting in increased cost and time consumption, and even irreparable losses.

Since the limitations in the existing upstream production of biomacromolecules make it difficult for enterprises to achieve high-quality, high-speed and high-output production as scheduled, it is urgent to find a solution for overcoming the impacts of metabolic products and product-related impurities generated in the upstream cell culture process on the production.

### SUMMARY

An object of the disclosure is to provide a method for producing a biomacromolecule by upstream staged retention, a production module used therein, and an application of the method/module in industrial production of polypeptides, fusion proteins and immunoglobulin molecules. The method provided herein optimizes the upstream process of the large-scale production of a recombinant protein, effectively improving the production efficiency, yield and purity.

Technical solutions of the disclosure are specifically described as follows.

In a first aspect, the present disclosure provides a staged-retention method for upstream production of a biomacromolecule, comprising:
(1) introducing a first retention system to a bioreactor to perform a first production stage; wherein the first retention system comprises a first filtering module; the first filtering module comprises a first filtering assembly having a pore size of 0.05-1 µm; and
(2) replacing the first retention system with a second retention system to perform a second production stage of the biomacromolecule; wherein the second retention system comprises a second filtering module; the second filtering module comprises a second filtering assembly having a molecular weight cut-off of 1/15-2/3 of a molecular weight of the biomacromolecule.

In an embodiment, the pore size of first filtering assembly is 0.1-0.5 µm, preferably 0.2-0.3 µm.

In an embodiment, the molecular weight cut-off of the second filtering assembly is 2/15-7/15, preferably 1/5-1/3, of the molecular weight of the biomacromolecule.

In an embodiment, the biomacromolecule has a molecular weight of 50-300 KD, preferably 100-250 KD, and more preferably 150-200 KD.

The first production stage corresponds to the initial proliferation stage of the cells, and may be referred to as the synthesis and optimization stage of the product. Due to the high culture temperature (37°C) in the initial environment, the cells proliferate rapidly, and a large number of cellular metabolic wastes, lactic acid products, and a small number impurities generated from the deamination or degradation of antibodies are accumulated. The second production stage is the product synthesis and collection stage, and it has been surprisingly found that after the metabolic products and impurities generated in the first production stage are removed, it is more conducive to the synthesis and collection of high-quality products in the second production stage. Therefore, a staged-retention system is creatively introduced to improve the quality and yield of the later products.

In an embodiment, the first retention system is mainly used to retain cells to remove metabolites and impurities; and the second retention system is mainly used to retain cells, enriches protein products and removes small molecule metabolites.

In an embodiment, in the upstream production of the biomacromolecule, the first production stage can be started as soon as the cells are seeded into the bioreactor.

In some embodiments, in the upstream production of the biomacromolecule, when the cell density reaches 6±1.5×10⁶ cells/mL, the first production stage is started.

In some embodiments, in the upstream production of the biomacromolecule, when the cell density reaches 10±5×10⁶ cells/mL, the first production stage is started.

In an embodiment, the cell density in the second production stage is not less than 15±5×10⁶ cells/mL, preferably not less than 35±5×10⁶ cells/mL.

In an embodiment, the cell density in the second production stage is 10×10⁶-30×10⁶ cells/mL.

In an embodiment, the cell density in the second production stage is 30×10⁶-50×10⁶ cells/mL.

In an embodiment, the cell density in the second production stage is 10×10⁶-300×10⁶ cells/mL. The cell fermentation broth is harvested to complete the second production stage until the cell viability drops to 50%, preferably 70%, and more preferably 80%; and most preferably 90%.

In an embodiment, the cell density in the second production stage is 30×10⁶-100×10⁶ cells/mL.

In an embodiment, the cell culture density of the second production stage keeps relatively stable, and its fluctuation range is not higher than 10×10⁶ cells/mL.

In an embodiment, the cell culture density of the second production stage shows an increasing trend with a fluctuation higher than 10×10⁶ cells/mL.

In an embodiment, the fluctuation range is 10×10⁶-290×10⁶ cells/mL.

In an embodiment, the fluctuation range is 10×10⁶-90×10⁶ cells/mL.

In an embodiment, the first and second retention systems are each independently an alternating tangential flow system, a tangential flow filtration system, a rotary filtration system, a deep-bed filtration system, a centrifuge system or a settling system.

In an embodiment, the bioreactor includes but is not limited to an air-lift bioreactor, a mechanically-agitated bioreactor, a bubble column bioreactor and a membrane bioreactor.

In an embodiment, the bioreactor includes but is not limited to a shaking incubator, a micro-bioreactor, a benchtop bioreactor, a stainless steel bioreactor, a glass bioreactor, a disposable bioreactor, a swinging bioreactor and multiple parallel tanks.

In an embodiment, the cells include, but are not limited to mammalian cells, plant cells, insect cells and microbial cells which are suitable as expression system for the industrial fermentation culture. In an embodiment, the cells are mammalian cells, where the mammalian cells include but are not limited to CHO cells, NSO cells, Sp2/0 cells, HEK cells and BHK cells.

In an embodiment, the cell culture is performed by perfusion culture, concentrated perfusion culture, fed-batch culture or concentrated fed-batch culture.

In an embodiment, the upstream production process of the biomacromolecule comprises:
(S1) activating the cells to be proliferated, and subjecting the cells to a step-wise scale-up culture to culture the cells in a bioreactor of a preset specification;
(S2) introducing the first retention system containing the first filtering module to cooperate with the bioreactor to perform the first production stage;
(S3) when the cell density reaches 15±5×10⁶ cells/mL, replacing the first retention system with the second retention system containing the second filtering module to perform the second production stage; and
(S4) monitoring the cell viability in real time; and collecting the cell fermentation broth for the downstream production process when the cell viability drops to no less than 50%, preferably 70%, and more preferably 80%, and most preferably 90%.

In an embodiment, when the cell density reaches 35±5×10⁶ cells/mL, the first retention system is replaced with the second retention system to perform the second production stage.

In an embodiment, when the cell density reaches 55±5×10⁶ cells/mL, the first retention system is replaced with the second retention system to perform the second production stage.

Since the downstream production and purification process is designed based on the characteristics of a specific biomolecular product, the production of biosimilars of herceptin is used as an example to illustrate the advantages of the staged-retention production provided herein in yield and quality.

In an embodiment, the production of biosimilars of avastin is used as another example to illustrate the advantages of the staged-retention production in yield and product quality.

In a second aspect, the disclosure provides a production module used in the upstream staged retention of the production of biomacromolecules, wherein the production module can be integrated into the retention systems and used in conjunction with the bioreactor; the production module comprises at least two filtering modules; the first filtering module comprises a first filtering assembly with a pore size of 0.05-1 µm, which is used to remove metabolic products and product-related impurities in the cell culture fluid; the second filtering module comprises a second filtering assembly having a molecular weight cut-off of 1/15-2/3 of the molecular weight of the biomacromolecule, which is used to harvest and enrich the biomacromolecule products.

In an embodiment, the pore size of the first filtering assembly is 0.1-0.5 µm, preferably 0.2-0.3 µm; and the molecular weight cut-off of the second filtering assembly is 2/15-7/15, preferably 1/5-1/3, of the molecular weight of the biomacromolecule.

In an embodiment, the impurity filtering module has a size corresponding to a diameter of a molecule of 20-50 KD, preferably 50 KD.

In an embodiment, the first and second filter assemblies are each independently hollow fiber-type, tube-type, roll-type or flat plate-type filter assembly.

In an embodiment, the first and second filter assemblies are each independently made of cellulose ester or non-cellulose ester, where the cellulose ester includes but is not limited to cellulose acetate, nitrocellulose and ethyl cellulose; the non-cellulose ester includes but is not limited to polysulfone (PS), polypropylene (PP), polyethylene (PE), polyvinyl chloride (PVC), polyethersulfone (PES) and polyvinylidene fluoride (PVDF).

In an embodiment, the bioreactor includes but is not limited to air-lift bioreactors, mechanically-agitated bioreactors, bubble column bioreactors and membrane bioreactors.

In an embodiment, the bioreactor includes but is not limited to a shaking incubator, a micro-bioreactor, a benchtop bioreactor, a stainless steel bioreactor, a glass bioreactor, a disposable bioreactor, a swinging bioreactor and multiple parallel tanks.

In an embodiment, the retention systems include but are not limited to alternating tangential flow systems, tangential flow filtration systems, rotary filtration systems, deep-bed filtration systems, centrifuge systems and settling systems.

In an embodiment, the cells include, but are not limited to mammalian cells, plant cells, insect cells and microbial cells which are suitable as expression system for the industrial fermentation culture. In an embodiment, the cells are mammalian cells, where the mammalian cells include but are not limited to CHO cells, NSO cells, Sp2/0 cells, HEK cells and BHK cells.

In a third aspect, the disclosure provides an application of the above production module/method in the industrial production of a biomacromolecule.

In an embodiment, the biomacromolecule is a polypeptide or a recombinant protein.

In an embodiment, the recombinant protein is an immunoglobulin or a genetically engineered antibody, and includes but is not limited to human-mouse chimeric antibodies, humanized antibodies, fully human antibodies, Fab fragments, F(ab')2, Fv, ScFv, single domain antibodies and bi/multi specific antibodies.

In an embodiment, the recombinant protein is a new protein formed through the fusion of a biologically active functional protein and the Fc fragment.

The module and method are applicable to microbial culture, medium, screening of clones or expression vectors, small-scale protein supply, process development, optimization and characterization, seed expansion and production.

### BRIEF DESCRIPTION OF THE DRAWINGS

Fig. 1 schematically shows the first stage of the upstream cell production.
Fig. 2 schematically shows the second stage of the upstream cell production.

### DETAILED DESCRIPTION OF EMBODIMENTS

In view of the problems of low efficiency, low yield and low purity in the existing upstream production process of biomacromolecules, the inventor, through extensive researches, has surprisingly found that the use of the staged-retention process in the upstream production process greatly optimizes the downstream production quality of the expressed protein, greatly improving the yield of the high-quality protein.

To facilitate understanding of the present disclosure, the primary terms of the present disclosure are defined as follows.

### SEC-HPLC

Size Exclusion-High Performance Liquid Chromatography is one of the most commonly-used methods for determining product purity, in which the purity is calculated according to the area normalization method. Components in the sample are divided into monomers, polymers (high molecular weight group) and fragments (low molecular weight group), and the larger the percentage of the monomer is, the higher the purity of the sample is.

### CEX-HPLC

Cation Exchange-High Performance Liquid Chromatography method is one of the most commonly-used methods for determining the charge purity of products, which can be used to reflect the uniformity of the products. According to the charge property, components in the sample are divided into acidic peak group, main peak group and alkaline peak group, where the higher the ratio of the acidic peaks and the alkaline peaks is, the higher the degree of charge heterogeneity of the products is, indicating the poor uniformity of the product.

### rCE-SDS

Reduced Capillary Gel-SDS electrophoresis adopts high-voltage electric field to provide driving force, where after reduced, the components, according to the molecular weight, are driven to move in the capillary tube at different speeds to be separated from each other according to the molecular weight. The higher ratio of the heavy chain and the light chain indicates a higher purity of the sample.

### nrCE-SDS

Non-reduced Capillary Gel-SDS electrophoresis also adopts high-voltage electric field to provide driving force, where the components, according to the molecular weight, are driven to move in the capillary tube at different speeds to be separated from each other. The ratio of the monomer is positively correlated with the purity of the product.

Cell viability/cell survival rate: the proportion of live cells to the total number of cells in a unit volume.

Since the antibody is susceptible to the effects of the culture temperature, culture time, stirring rate and pH during the fermentation, various post-translational modifications and changes such as deamination, oxidation, glycation and N-terminal pyroglutamate cyclization will occur, which will affect the charge distribution on the antibody. Whether a post-translational modification is a key quality attribute or not is dependent on whether it occurs at a crucial position (for example, occurring in CDR region to affect the function and at FcRn binding site to affect PK). At the same time, charge distribution/charge purity is a very sensitive quality attribute and can be used as a desirable indicator for the uniformity evaluation. However, the control of charge heterogeneity has always been the focus and difficulty in the production of antibody drugs. Compared to the prior art, the disclosure has advantages of maintaining a higher charge purity of the product to reduce the occurrence of post-translational modifications and degradation which may affect charge and simultaneously further ensuring the similarity of the charge distribution among different batches in large-scale production. Moreover, substances generated during the cell proliferation phase, such as ammonium ions, proteases, other host proteins and DNA, may affect cell growth and product quality (for example, some enzymes product degradation caused by certain), meanwhile, the presence of these impurities in the harvested fluid will greatly affect the downstream purification yield, and the yield and quality of the product due to the failure in complete removal.

In addition, the system and method of present disclosure can further solve the problems of unsatisfactory product quality and uniformity in the existing feed culture system. In the large-scale antibody production, since the fed-batch fails to ensure the stable impurity control, it is often switched to the perfusion culture, resulting in increased complexity of the process. The present disclosure can solve the problems of instability and high content of product-related impurities in fed-batch mode, and can produce high-quality products without the need for process switching, promoting the development of large-scale antibody production.

Therefore, compared with the prior art, the present disclosure uses a first filtering assembly suitable for discharging impurities and cell metabolites and a second filtering assembly suitable for collecting and enriching the desired product in sequence in the process of cell culture, ensuring the large-scale and high-yield production of antibody products in a relatively short period of time under relatively fixed cell passage, density and viability conditions in the product retention stage. The major beneficial effects are listed as follow.
(1) The disclosure improves the product uniformity, solving the problem of charge heterogeneity in the antibody production.
(2) The disclosure can effectively remove metabolites produced in the cell proliferation stage, especially high molecular weight proteasome, reducing the impact of these metabolites on the product during the retention stage, and improving product purity and quality.
(3) The disclosure can remove the small number of antibody products of which the occurrence is caused by the relatively high incubation temperature in the early stage of cell proliferation. These early products are prone to degradation under relatively high temperature conditions and will enter the final product as product-related impurities. Therefore, the present disclosure is beneficial to further reduce product-related impurities and ensure high yield and high purity of the final product.
(4) In the disclosure, the production cycle is shortened, and the downstream production process is simplified, thus lowering the production cost.

The disclosure will be further illustrated below with reference to embodiments. It should be understood that these embodiments are merely illustrative of the present disclosure, and are not intended to limit the scope of the disclosure. Unless otherwise specified, the experiments in the following examples are generally performed in accordance with the conventional conditions or the basic test methods in the third edition of the "Molecular Cloning: A Laboratory Manual " or the conditions recommended by the manufacturer.

### Example 1 200 L bioreactor culture based on staged retention

### Antibody

The antibody prepared in this example was a biosimilar developed according to the commercially available therapeutic antibody Herceptin. The expression cell line was Chinese Hamster Ovary Cell (CHO), and the monoclonal antibody was IgG1/kappa having the same amino acid sequence as the original drug Herceptin (Herceptin®) (INN trastuzumab, IMGT number: 7637); and its molecular weight was 145 kDa.

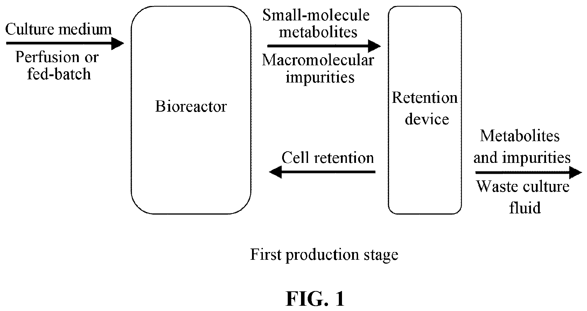

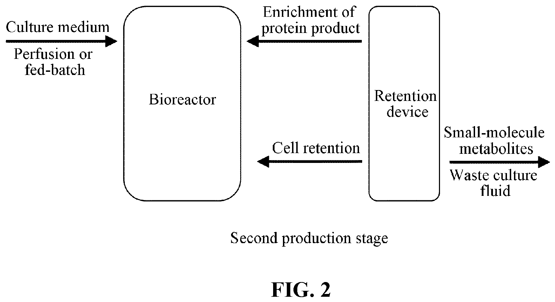

The procedures of the upstream first production stage were schematically shown in Fig. 1, and the procedures of the upstream second production stage were schematically shown in Fig. 2.

### Cell thawing

The cell cryopreservation tube was taken out and directly immersed in a water bath at 37°C under frequent shaking to thaw the cells as soon as possible. The cryopreservation tube was transferred from the 37°C water bath, sterilized with 75% alcohol and opened in the clean bench. The cell suspension was pipetted with a sterile pipette to a 15 mL centrifuge tube containing 1 mL of pre-incubated CD FortiCHO™ Medium and shaken uniformly. 30-60 s later, the centrifuge tube was added with 2 mL of pre-incubated basic medium and shaken uniformly. 30-60 s later, the centrifuge tube was added with 4 mL of pre-incubated basic medium and shaken uniformly. 30-60 s later, the centrifuge tube was centrifuged at 700 rpm for 5 min, and the supernatant was discarded. The cells were suspended with 2 mL of pre-incubated basic medium, transferred to a cell culture flask containing 8 mL of basic medium and shaken uniformly.

### Expansion culture

The cells were counted and subjected to expansion culture once every other 2 days, where the medium was CD OptiCHO™ medium (Thermo Fisher Scientific), and the initial cell density after the expansion was returned to 6-10×10⁵ cells/mL. The expansion culture was continued until the expansion volume met the requirements of 10 L reactor inoculation, i.e., the cell density was 2.5-4.5×10⁶ cells/mL, and the survival rate was ≥90%. The specific control parameters were shown in Table 1.

**Table 1 Conditions of the expansion culture**

| Shaking speed | Temperature | Carbon dioxide concentration |
|---|---|---|
| 120 rpm | 37°C | 5% |

After inoculated into 10 L WAVE bioreactor, the cells were sampled every day, and the specific control parameters were shown in Table 2.

**Table 2 Incubation conditions**

| Temperature | pH | Dissolved Oxygen | Rotating speed | Swinging angle | Ventilation |
|---|---|---|---|---|---|
| 37°C | 7 | 50% | 25 rpm | 8° | 0.2 L/min |

After cultured for 3 d, the cells were further transferred to be cultured in a 50 L bioreactor, and the specific control parameters were shown in Table 3.

**Table 3 Incubation conditions in the 50 L bioreactor**

| Temperature | pH | Dissolved Oxygen | Rotating speed | Culture time |
|---|---|---|---|---|
| 37°C | 7 | 50% | 90 rpm | 3d |

### Perfusion culture through the combination of ATF with 200 L bioreactor

In this example, a 200 L-alternating tangential flow system from Refine Techology, LLC, was used. The hollow fiber column in the ATF device and the diaphragm at the top of the base were assembled, and then the ATF device was connected to the vacuum pump and the controller. The vacuum fiber membranes respectively with a pore size of 0.2 µm and a pore size for retaining 50 KD substances were respectively installed in two ATF systems (hereinafter respectively abbreviated as 0.2 µm ATF system and 50 KD ATF system), rinsed with 25% ethanol for 10 min, then repeatedly rinsed with deionized water to remove residual ethanol, and then sterilized in a high-temperature moist-heat sterilizer for 45 min for use.

The 0.2 µm ATF system was connected to the 200 L bioreactor. After the cells were cultured in 50 L bioreactor for 3 d, the cells were inoculated into the 200 L bioreactor with an initial cell density of 1×10⁶ cells/mL, and the specific control parameters were shown in Table 4.

**Table 4 Incubation conditions in 200 L bioreactor**

| Culture temperature | pH | Dissolved Oxygen | Rotating speed |
|---|---|---|---|
| 37°C | 7 | 50% | 90 rpm |

When the cell density grew to 10×10⁶ cells/mL, the ATF system was connected, and the controller and vacuum pump in the ATF device were turned on. The volume of the medium required to be replaced every day was calculated according to the cell growth amount and the cell growth rate, regulating the speed at which the fresh culture medium was fed and the speed at which the waste culture medium was discharged. When the cell density grew to 40-50x 10⁶ cells/mL, the 0.2 µm ATF system was replaced with the 50 KD ATF system, and the controller and vacuum pump in the ATF device were continued to be turned on to perform the second production stage. In an embodiment, before the cell density reached 40-50×10⁶ cells/mL, the bioreactor was controlled to have a culture temperature of 34°C, pH of 6.9, a dissolved oxygen content of 50% and a rotating speed of 125 rpm (the results obtained based on these conditions were shown in Table 6). In an embodiment, the temperature and other culture conditions of the bioreactor can be adjusted simultaneously with the replacement of the 0.2 µm ATF system with the 50 KD ATF system. When the cell density reached approximately 100×10⁶ cells/mL, the supernatant was collected.

### Cell clarification

The clarification of cell culture fluid was generally performed by centrifugation or microfiltration combined with deep-bed filtration to complete the removal of cells and cell debris from the culture fluid. In this example, the cell suspension from the 200 L bioreactor was centrifuged at 6,000×*g* and 4°C for 15 min, and the cells were removed and the fermentation supernatant was collected. Then the fermentation supernatant was treated using the deep-bed filtration method, which was currently commonly used for the clarification of cell culture fluid. In the deep-bed filtration method, a loose and porous cellulose skeleton structure with diatomaceous earth filled therein was adopted, where the surface of the inlet end of the structure was provided funnel-shaped pores. The above-mentioned structure eliminated the shortcomings in the traditional microfiltration that the surface structure was prone to blockage and the filtration efficiency was low, and had the advantage of retaining small-volume cell debris compared to the centrifugation. In addition, based on the adsorption of diatomaceous earth, DNA and HCP can be trapped. In this example, MX0HC10FS1 depth filter from Millipore was used, and after the container storing the fermentation supernatant was connected to the peristaltic pump (any commercially available peristaltic pumps meeting the flow rate requirement were applicable), the depth filter membrane was rinsed with 10 CV of ultrapure water. Then 1 CV of the deep filtration buffer containing 25 mM Tris-HCl and 100 mM NaCl (pH 7.4±0.2) was used to rinse the depth filter membrane at a flow rate of 100-300 LMH. The fermentation supernatant was filtered at a flow rate of 100-300 LMH, and the filtrate was collected. After the sample was completely loaded, the filter membrane was washed with 2 CV of the deep filtration buffer, and the filtrate was collected. The pre-membrane pressure during the process was controlled to be 2 bar or less.

### Protein A affinity chromatography

The protein A affinity chromatography played a role in capturing host proteins, endotoxins and DNA in the antibody purification. In this example, the filler of the protein A affinity chromatography was Mabselect Sure (manufacturer: GE). The filtrate obtained from the deep-bed filtration was loaded at a flow rate of 300 cm/h, where a ratio of the loading amount (mg) to the filler (mL) was guaranteed to be less than 35 mg/mL. After the sample was completely loaded, the chromatography column was washed with 5 CV of a washing buffer containing 25mM Tris-HCl and 50mM NaCl (pH 7.4) at a flow rate of 300 cm/h. Then the chromatography column was eluted with an eluent containing 100 mM acetic acid-sodium acetate (pH 3.6±0.2, conductivity 0.9±0.2 mS/cm) at a flow rate of 300 cm/h. During the elution process, the eluate was monitored at 280 nm in real time, and when the absorbance rose to 0.1 AU, the eluate was started to be collected, and when the absorbance decreased to 0.5 AU, the collection was terminated. Three cycles of protein A affinity chromatography were performed.

### Virus inactivation and deep-bed filtration of inactivated viruses

In this example, the inactivation of retroviruses and enveloped viruses was completed through the incubation with a low pH eluate obtained from the affinity chromatography. The eluate was adjusted to pH 3.6-3.8 with 10% acetic acid and the viruses were inactivated for 60-120 min.

The inactivated virus sample was filtered with MX0HC10FS1 depth filter from Millipore. Specifically, the depth filter membrane was washed sequentially with 10 CV of ultrapure water and 1 CV of 50 mM acetic acid-sodium acetate buffer (pH 5.2), and then the pipelines were connected for sample filtration, where the flow rate was 100-300 LMH and the filtration pressure was less than 2 bar. After the sample was loaded, the depth filter membrane was washed sequentially with 1 CV and 2 CV of the 50 mM acetic acid-sodium acetate buffer (pH 5.2), and the filtrate was collected.

### Cation exchange chromatography

To further remove impurities, DNA and endotoxins from the product, three cycles of cation exchange chromatography were adopted in the deep-bed filtration of inactivated viruses. The Fractogel EMD COO-(M) (manufacturer: Merck) was selected as the filler of cation exchange chromatography. The chromatographic column was equilibrated with 3 CV of the equilibration buffer (50 mM acetic acid-sodium acetate, pH 5.2). The sample was loaded at a flow rate of 120 cm/h, where a ratio of the loading amount (mg) to the filler (mL) was guaranteed to be less than 45 mg/mL. After the sample was completely loaded, the chromatography column was washed with 5 CV of the equilibration buffer at a flow rate of 180 cm/h. Then the gradient elution was carried out using 10 CV of an eluent, where the eluent was composed of the equilibration buffer (gradually decreasing from 100% to 0) and a buffer (gradully increasing from 0 to 100%) containing 50 mM acetic acid-sodium acetate and 300 mM sodium chloride (pH 5.2); the flow rate was 180 cm/h. The eluate was collected during the elution process.

### Anion exchange chromatography

Trace impurities in the sample such as various viruses, DNA and endotoxin can be effectively removed using anion exchange chromatography. The anion exchange chromatography generally adopted a flow-through mode, where the impurities will bind to the filler while the target protein (such as monoclonal antibodies) directly flowed out through the chromatography column without binding to the filler due to its high isoelectric point. Capto Q (manufacturer: GE) was used in anion exchange chromatography in this example. The chromatography column was equilibrated with 2 CV of a pre-equilibrium solution (50 Mm Tris-HCl + 1M NaCl, pH 8.0), and then equilibrated with 3CV of an equilibrium solution (50 mM Tris-HCl, pH 8.0). The sample was loaded at a flow rate of 180 cm/h, where a ratio of the loading amount (mg) to the filler (mL) was kept below 40 mg/mL. The eluate was collected when the UV280 reading increased to 0.1 AU. After the sample loading was completed, the chromatography column was washed with 3 CV of the equilibrium solution at a flow rate of 180 cm/h, and the collection was terminated when the UV280 reading decreased to 0.1 AU.

### Virus filtration

The virus filtration can be used to remove non-enveloped viruses. In the production process, 1.02 m²/batch of virus filter membrane was used for filtration. The pressure tank was used, treated with 0.5M NaOH, washed with water by injection and then communicated with compressed air for filtration. In this example, VPMG201NB1 (manufacturer: Merck Millipore) was used for deep-bed filtration. Before use, the depth filter membrane was rinsed sequentially with 10 CV of ultrapure water and 2 CV of a 500 mM acetic acid-sodium acetate buffer (pH 5.2). After that, the pipelines were connected for sample filtration, where the flow rate was 100-300 LMH and the filtration pressure was 1.9-2.0 bar. After the sample was loaded, the depth filter membrane was washed sequentially with 1 CV and 2 CV of the 50 mM acetic acid-sodium acetate buffer (pH 5.2), and the filtrate was collected.

### Fluid replacement by ultrafiltration

The ultrafiltration membrane with a pore size of 30 KD and model number of P2C030C25 (manufacturer: Merck Millipore) was cleaned, and washed with 20 mM sodium phosphate buffer (pH 6.0) until the post-membrane pH was 6.0. The sample was pumped into the ultrafiltration membrane with the peristaltic pump, where the inlet flow rate was controlled to be 200 LMH, and the transmembrane pressure was controlled to 0.35 bar. The 20 mM sodium phosphate buffer (pH 6.0) was used for fluid replacement to harvest the antibody product.

### Comparative example 1 Cell culture in 200 L bioreactor based on single-retention method

The operation process provided herein was basically the same as Example 1, and the difference was merely that when the cell density grew to 10×10⁶ cells/mL, the ATF system was connected, and the controller and vacuum pump in the ATF device were turned on. The ATF system used herein included a vacuum fiber membrane module with a retention molecular weight of 50 KD. The fermentation was completed until the cell density grew to about 100×10⁶ cells/mL. The subsequent purification was the same as Example 1.

After the protein A affinity chromatography was completed, the samples obtained in Example 1 and Comparative Example 1 were loaded for CEX-HPLC analysis to determine the content of charge variants, where the distribution of the acidic and alkaline peaks was shown in Table 5.

**Table 5 Peak distribution by CEX-HPLC analysis**

| CEX-HPLC | | | |
|---|---|---|---|
| Batch | Acidic peak | Neutral peak | Alkaline peak |
| Staged-retention method (Example 1) | 24.9 | 67.8 | 7.3 |
| Single-retention method (Comparative Example 1) | 30.7 | 61.8 | 7.5 |

It can be seen from Table 5 that compared to the antibody products obtained by the single-retention method, the antibody products obtained by the staged-retention method had better charge purity, higher level of neutral peak and lower level of acidic peak.

The effects of the two upstream production processes on the final products were compared after the purification was completed. It can also be found that the ratio of acid peak of the final product produced by the single-retention method had been improved to a certain extent, but not very significant, which was reflected in the biological activity. The products obtained by the staged-retention method showed significantly better biological activity than the products obtained by the single-retention method. At the same time, the staged-retention method can result in higher yield of qualified final products compared to the single-retention method. The results were shown in Table 6.

**Table 6 Properties of the products obtained by single-retention method and staged-retention method**

| Upstream production method | | Single-retention method (Comparative Example 1) | Staged-retention method (Example 1) |
|---|---|---|---|
| Cell culture stage | | 12 g/L | 12 g/L |
| Final product yield | | 6 g/L | 9.2 g/L |
| Character/color | | Light yellow liquid (Y5) | Light yellow liquid (Y5) |
| pH | | 6.0 | 5.9 |
| Osmotic pressure | | 63 | 63 |
| SEC-HPLC | Polymer | 0.8 | 0.6 |
| | Main peak | 99.2 | 99.4 |
| | Fragment | 0 | 0 |
| Reduced CE-SDS purity (light chain + heavy chain) | | 99.3 | 99.1 |
| Non-reduced CE-SDS purity (monomer) | | 97.1 | 97.3 |
| CEX-HPLC | Acidic peak | 29.3 | 22.7 |
| | Neutral peak | 63.2 | 67.8 |
| | Alkaline peak | 7.5 | 9.5 |
| Biological activity | | 94% | 103% |

### Example 2 Cell culture in 2 L bioreactor based on staged retention Antibodies

The antibody prepared herein was a recombinant anti-vascular endothelial growth factor (VEGF) humanized monoclonal antibody, which was obtained by fermentative expression in mammalian cells *in vitro.* The animal cells were Chinese hamster ovary (CHO) cells, and the monoclonal antibody was IgG1/kappa with an amino acid sequence the same as the original drug Avastin® (INN: bevacizumab, IMGT number: 8017, http://www.imgt.org/mAb-DB/) and a molecular weight of 149 kDa.

### Cell thawing and ATF perfusion culture in 2 L bioreactor

The procedure for cell thawing was the same as in Example 1.

Seed cells were cultured using a conventional cell culture process, specifically, when the inoculation requirements were met, that was, the cell density reached 4.0-6.5×10⁶ cells/mL and the viability was ≥90%, the seed cells were inoculated aseptically into a 2 L bioreactor containing CD FortiCHO™ Medium for culturing. After inoculation, the cell density was 1.0×10⁶ cells/mL, and the viability was ≥90%. The initial cell culture conditions were described as follows: culture temperature 37°C; stirring speed: 95 rpm; pH: 7.20±0.05; and dissolved oxygen: 50%.

An ATF system from Refine Technology was used. The hollow fiber column in the ATF device and the diaphragm at the top of the base were assembled, and then the vacuum pump and controller were connected. The pipe connecting the top of the hollow fiber column with a pore size of 0.2 µm to the reactor was installed. When the cells were cultured in the bioreactor to a density of 4.5-5.5×10⁶ cells/mL, the vacuum pump and controller in the ATF device were turned on, where the volume of the medium required to be replaced per day was determined based on the cell growth amount and the cell growth rate, adjusting the rate at which the fresh medium was fed and the rate at which the culture waste was discharged. When the perfusion volume reached the preset value every day, if glucose was insufficient, an additional 30% glucose solution was appropriately supplemented. When the cell density reached 65±h×10⁶ cells/mL, the culture temperature was reduced to 32°C and pH was reduced to 7±0.05. When the cell density was further increased to 95±5×10⁶ cells/mL, the original 0.2 µm hollow fiber membrane was replaced with the treated sterile 50 KD vacuum fiber membrane to perform the second production stage. The fluctuation of the cell density was maintained within 10×10⁶ cells/mL. When the cell viability decreased to 90%, the culture in the bioreactor was terminated, and the cell culture fluid was collected.

In an embodiment, the cell culture fluid was collected when the cell viability dropped to 80%. In an embodiment, the cell culture fluid was collected when the cell viability dropped to 70%. In an embodiment, the cell culture fluid was collected when the cell viability dropped to 50%.

### Clarification

In this example, after the depth filter membrane (D0HC (number MD0HC10FS1, manufacturer: Merck)) was connected to the peristaltic pump (any commercially available peristaltic pumps meeting the flow rate requirement were applicable), 10 CV of ultrapure water was pumped to wash the depth filter membrane. Then the depth filter membrane was washed with 1 CV of the deep-bed filtration buffer containing 25 mM Tris-HCl and 100 mM NaCl (pH 7.4±H.2) at a flow rate of 100-300 LMH. Then the culture fluid was filtered at a flow rate of 100-300 LMH and the filtrate was immediately collected. After that, the depth filter membrane was washed with 2 CV of the deep filtration buffer containing 25 mM Tris-HCl and 100 mM NaCl (pH 7.4±0.2) and the filtrate was collected, where the pre-membrane was controlled to be no more than 2 bar.

### Protein A affinity chromatography

The packing material used in the protein A affinity chromatography was JWT 203 (manufacturer: JSR). The filtrate obtained by the deep-bed filtration was loaded at a flow rate of 300 cm/h, where a ratio of the loading amount (mg) to the packing material as guaranteed to be less than 35 mg/mL. After the sample was loaded, the chromatography column was washed with 5 CV of a washing buffer containing 25 mM Tris-HCl and 50 mM NaCl (pH 7.4) at a flow rate of 300 cm/h, and then eluted with a 100 mM acetic acid-sodium acetate eluent (pH 3.6±0.2, conductivity 0.9±0.2 mS/cm) at a flow rate of 300 cm/h. The eluate was monitored at 280 nm in real time. When the UV280 reading rose to 0.5 AU, the eluate was started to be collected, and when the UV280 reading decreased to 0.5 AU, the collection was terminated. The collected sample was adjusted to a pH value of 3.5-3.7 at room temperature, and used to treat viruses for 1-2 hours for inactivation.

### Anion exchange chromatography

The crude sample was purified by pure chromatography to prepare the stock solution. Capto Q packing material (manufacturer: GE healthcare) was used in the anion exchange chromatography. The chromatography column was equilibrated sequentially with 2 CV of a pre-equilibrium solution (50Mm Tris-HCl + 1M NaCl, pH 8.0), and 3 CV of an equilibrium solution (50 mM Tris-HCl, pH 8.0). The sample was loaded at a flow rate of 180 cm/h. The elution peak was collected when the UV280 reading increased to 0.1 AU, and a ratio of the loading amount (mg) to the packing material (mL) was kept below 40 mg/mL. After the sample loading was completed, the chromatography column was washed with 3 CV of the equilibrium solution at a flow rate of 240 cm/h. The collection was terminated when UV280 reading decreased to 0.1 AU.

### Cation exchange chromatography

The collected sample was further treated by cation exchange chromatography. The Fractogel EMD COO-(M) exchanger (manufacturer: Merck) was selected for the cation exchange chromatography. The chromatographic column was equilibrated with 3 CV of the equilibration solution (50 mM acetic acid-sodium acetate, pH 5.2). The GB222 sample was loaded at a flow rate of 180 cm/h, where a ratio of the loading amount (mg) to the packing material was guaranteed to be less than 45 mg/mL. After the sample was loaded, the column was washed with 5 CV of the equilibrium liquid at a flow rate of 180 cm/h. Then the column was subjected to gradient elution with 10 CV of an eluent, where the eluent was composed of a buffer (gradually increasing from 0 to 100%) containing 50 mM acetic acid-sodium acetate and 300 mM sodium chloride (pH 5.2) and the equilibrium solution (gradully decreasing from 100% to 0), and loaded at a flow rate of 180 cm/h. The eluate was collected during the elution process.

### Fluid exchange by ultrafiltration

The purified sample was subjected to fluid exchange by ultrafiltration. The ultrafiltration membrane with a model number of P2C030C25 and pore size of 30 KD (manufacturer: Merck Millipore) was cleaned and rinsed with 20 mM sodium phosphate buffer (pH 6.0) until pH of the post-membrane was 6.0. The sample was pumped into the ultrafiltration membrane by the peristaltic pump for ultrafiltration and concentration, where the inlet flow rate was controlled to be 300 LMH and the transmembrane pressure was controlled to 0.3-0.7 bar. The sample was concentrated to about 20 mg/mL through the first ultrafiltration, then a 20 mM sodium phosphate buffer (pH 6.0) was used for fluid exchange, where the sodium phosphate buffer was 10 times as much as the sample in volume. After that, the ultrafiltration membrane was emptied to obtain the stock solution.

### Comparative Example 2 Antibody Production in 2 L-ATF system based on single-retention method

The ATF system from Refine Technology was also used herein. First, the hollow fiber column in the ATF device and the diaphragm at the top of the base were assembled, and then the vacuum pump and controller were connected. The pipe connecting the top of the hollow fiber column with a pore size of 50 KD to the reactor was installed, placed in 25% ethanol, and sterilized according to the method of Example 1.

The cell culture was basically the same as Example 2, and the main difference was that when the cell density grew to 4.5-7.5×10⁶ cells/mL, the ATF system was connected, and the controller and vacuum pump in the ATF device were turned on. The ATF system used herein included a vacuum fiber membrane assembly with a retention molecular weight of 50 KD. The culture was terminated until the cell viability dropped to 90%. The subsequent purification steps were the same as Example 2.

The cell culture fluid was taken for preliminary analysis on the 10^{th} day of the single-retention cell culture. The results showed that the acidic peak content was about 26%, by contrast, the acidic peak was only about 10% on the 10^{th} day of the staged-retention culture. After the subsequent purification was completed, the acidic peak content in the final product obtained by the staged-retention production was also lower than that in the final product obtained by the single-retention production. After ultrafiltration and fluid exchange, the requirement for SEC-HPLC was that the purity of the antibody monomer was ≥ 95%, while for the three batches of products obtained by single-retention method, the detected purities of the antibody monomer were only 93%, 92.8% and 93%, which was much lower than the purity of the product obtained by the staged-retention method of Example 2. Therefore, in addition to the yield, the staged-retention process was significantly superior to the single-retention method in the charge purity and molecular size purity. The further analysis and comparison results of the products respectively obtained by the above retention methods were shown in Table 7.

**Table 7 Comparison of parameters of products respectively obtained by single-retention and staged-retention**

| Upstream production method | | Single-retention method | Staged-retention method |
|---|---|---|---|
| Character/color | | Light brown liquid (B6) | Light brown liquid (B6) |
| pH | | 6.1 | 6.2 |
| Osmotic pressure | | 280 | 272 |
| SEC-HPLC | Polymer | 6.2 | 3.5 |
| | Main peak | 93.7 | 96.5 |
| | Fragment | 0 | 0 |
| Reduced CE-SDS purity | | 97.3 | 98.2 |
| Non-reduced CE-SDS purity | | 96.7 | 97.8 |
| CEX-HPLC | Acidic peak | 31.7 | 21.9 |
| | Neutral peak | 57.2 | 68.8 |
| | Alkaline peak | 11.2 | 9.3 |
| Biological activity | | 99% | 104% |

### Example 3 Staged-retention upstream production under different cell densities

It had been further found that the applicable cell density of the staged-retention process can be as low as 15±5×10⁶ cells/mL. For example, under low-density cell culture conditions, the second production stage can be started when the cell density reached 15±5×10⁶ cells/mL, preferably 35±5×10⁶ cells/mL. When the second production stage was started under the above cell density conditions, the final product was still significantly better than that obtained by traditional single-retention in the yield and the charge purity. The antibody of Example 2 was used as an example, and the comparison between the single-retention method and staged-retention method under cell densities respective of 15±5×10⁶ cells/mL (ranging from 10×10⁶ cells/mL to 20×10⁶ cells/mL) and 35±5×10⁶ cells/mL (ranging from 30×10⁶ cells/mL to 40×10⁶ cells/mL) was shown in Table 8.

**Table 8 Comparison of single retention and staged retention under different cell densities**

| Cell density | 15±5×10⁶ cells/mL | | 35±5× 10⁶ cells/mL | |
|---|---|---|---|---|
| Production condition | Single-retention method | Staged-retention method | Single-retention method | Staged-retention method |
| SEC-HPLC main peak | 91% | 95% | 93% | 96% |
| CEX-HPLC acidic peak | 31% | 20% | 26% | 17% |

### Example 4 Applicability of products of different molecular weights

The staged-production process of the present disclosure was also suitable for the production of protein products of different molecular weights. The recombinant genetically engineered antibodies were used as an example, where the staged-retention process was applicable both in the production of antibody Fab region of an average molecular weight of about 50 KD and the production of bi/multi-specific antibodies having a molecular weight of nearly 300 KD. In particular, in the production of bi/multi-specific antibodies, since these antibodies had complex structures and often involved specially designed mutations, product-related impurities will be probably generated. In the use of the staged-retention process, the product-related impurities will pass through the larger filter holes to be discharged in the first production stage, which was conducive to the subsequent purification and development. Due to the large difference in molecular weight of protein products, the present disclosure further specified the size of filter pores of the filter assemblies used in the two production stages. The filter assembly used in the first production stage had a pore size of 0.05-1 µm, which can allow the small molecule metabolites and large molecule product-related impurities to be removed. In a preferred example, the pore size of the filter assembly was 0.1-0.5 µm, which can more effectively improve the removal efficiency and product yield. The filter assembly used in the second production stage had a molecular weight cut-off of 1/15-2/3 of the molecular weight of the product, which can ensure desirable enrichment of the product. When the molecular weight cut-off was 2/15 to 7/15 of the product molecular weight, the yield was higher, and when the molecular weight cut-off was 1/5 to 1/3 of the product molecular weight, the yield of the enriched antibodies reached the highest.

In this example, the single-retention method and the staged-retention method were respectively used to express and purify the protein fragments of the Fab region (∼50 KD) of the Herceptin antibody sequence in Example 1. The ATF-2L system from Refine Technology was used in the staged-retention method. A hollow fiber column with a pore size of 0.1 µm was used in the first stage of culture. When the cell density reached 10-15×10⁶ cells/mL, the 0.1 µm hollow fiber membrane was replaced with the pre-treated sterile 10 KD vacuum fiber membrane to perform the second production stage. For the single-retention method, a 10 KD vacuum fiber membrane was installed in the ATF system at the beginning. Under the same culture conditions, compared to the single-retention method, the staged-retention method can achieve a 4% increase in the yield of the Fab region of Herceptin antibody sequence, and a 2% increase in purity. Moreover, other protein fragments with a molecular weight of 50 KD were also treated using the above methods, and the results also showed that compared to the traditional single-retention method, the staged-retention method can effectively increase the yield by an average of 2%-4% and the purity by an average 1%-2%.

In this example, the single-retention method and the staged-retention method were also adopted to express and purify the bispecific antibody, where the bispecific antibody was formed by directly ligating the C-terminus of the Fab region (∼50 KD) of the Herceptin antibody sequence in Example 1 to the N-terminus of the variable region of the original drug Avastin antibody in Example 2, and had a symmetrical structure and a molecular weight of about 250 KD. In the staged-retention method, the ATF-2L system from Refine Technology was still used. The hollow fiber column with a pore size of 0.5 µm was used to achieve the culture in the first stage, and when the cell density reached 30-40×10⁶ cells/mL, the 0.5 µm hollow fiber membrane was replaced with the aseptically-treated 50 KD vacuum fiber membrane to perform the second production stage. In the single-retention method, the 50 KD vacuum fiber membrane was installed in the ATF system at the beginning. Under the same culture conditions, compared to the single-retention method, due to the complexity of the bispecific antibody, the staged-retention method can remove metabolites, polymers and antibody molecules with poor quality at the early stage of the cell culture in the first production stage. Therefore, the staged-retention method can achieve a 10% increase in the yield of bispecific antibody sequences and 5% increase in purity. Other protein fragments with a molecular weight of 250 KD were also treated by such two methods for comparison, and the results showed that compared to the traditional single-retention method, the staged-retention method can effectively increase the yield by an average of 5-10% and the purity by an average of 3-5% in the preparation of large-molecular-weight bispecific or trispecific antibodies.

Described above are merely preferred embodiments of the disclosure, which are not intended to limit the disclosure. It should be understood that the upstream staged-retention production process can be integrated into the culture in various forms of bioreactors, or used in various existing retention systems, such as tangential flow filtration (TFF) systems, rotary filtration systems, depth filtration systems, centrifugation systems and settling systems. Various variations and modifications made without departing from the spirit of the disclosure should fall within the scope of the disclosed defined by the appended claims.

## Claims

1. A staged-retention method for upstream production of a biomacromolecule, comprising:
(1) introducing a first retention system to a bioreactor to perform a first production stage; wherein the first retention system comprises a first filtering module; and the first filtering module comprises a first filtering assembly having a pore size of 0.05-1 µm; and
(2) replacing the first retention system with a second retention system to perform a second production stage; wherein the second retention system comprises a second filtering module; and the second filtering module comprises a second filtering assembly having a molecular weight cut-off of 1/15-2/3 of a molecular weight of the biomacromolecule.

2. The staged-retention method according to claim 1, **characterized in that** the pore size of the first filtering assembly is 0.1-0.5 µm; and/or
the molecular weight cut-off of the second filtering assembly is 2/15-7/15 of the molecular weight of the biomacromolecule.

3. The staged-retention method according to claim 2, **characterized in that** the pore size of the first filtering assembly is 0.2-0.3 µm; and/or
the molecular weight cut-off of the second filtering assembly is 1/5-1/3 of the molecular weight of the biomacromolecule.

4. The staged-retention method according to any one of claims 1-3, **characterized in that** the biomacromolecule has a molecular weight of 50-300 KD.

5. The staged-retention method according to claim 4, **characterized in that** the molecular weight of the biomacromolecule is 100-250 KD or 150-200 KD.

6. The staged-retention method according to claim 1, **characterized in that** a cell density in the second production stage is not lower than 15±5×10⁶ cells/mL.

7. The staged-retention method according to claim 6, **characterized in that** a cell density in the second production stage is not lower than 35±5×10⁶ cells/mL.

8. The staged-retention method according to claim 6, **characterized in that** a fluctuation in the cell density during the second production stage is not higher than 10×10⁶ cells/mL, or the cell density increases progressively in the second production stage with a fluctuation higher than 10×10⁶ cells/mL.

9. The staged-retention method according to claim 1, **characterized in that** the first and second retention systems each are an alternating tangential flow system, a tangential flow filtration system, a rotary filtration system, a deep-bed filtration system, a centrifuge system or a settling system; and/or
the bioreactor is an air-lift bioreactor, a mechanically-agitated bioreactor, a bubble column bioreactor or a membrane bioreactor; and/or
the cells are mammalian cells, plant cells, insect cells or microbial cells, preferably mammalian cells.

10. The staged-retention method according to claim 1, **characterized in that** the upstream production of the biomacromolecule is performed by perfusion culture, concentrated perfusion culture, fed-batch culture or concentrated fed-batch culture.

11. The staged-retention method according to claim 1, **characterized in that** the biomacromolecule is a polypeptide or a recombinant protein.

12. The staged-retention method according to claim 11, **characterized in that** the recombinant protein is a genetic engineering antibody, a functional protein with biological activity or a protein formed from the fusion of the Fc fragment.

13. A production module used in the staged-retention method according to claim 1, comprising: the first filter module and the second filter module;
wherein the first filtering module comprises the first filtering assembly having a pore size of 0.05-1 µm; the second filtering module comprises the second filtering assembly having a molecular weight cut-off of 1/15-2/3 of the molecular weight the biomacromolecule; the first filtering module and the second filtering module are respectively placed in the first and second retention systems, or applied in the same retention system via replacement.

14. The production module according to claim 13, **characterized in that** the pore size of the first filtering module is 0.2-0.3 µm; and/or
the molecular weight cut-off of the second filtering module is 1/5-1/3 of the molecular weight of the biomacromolecule.

15. The production module according to claim 14, **characterized in that** the pore size of the first filtering module is 0.2-0.3 µm; and/or
the molecular weight cut-off of the second filtering module is 1/5-1/3 of the molecular weight of the biomacromolecule.

16. An application of the staged-retention method according to any one of claims 1-12 or the production module according to any one of claims 13-15 in the upstream production of a biomacromolecule, **characterized in that** the biomacromolecule is a protein product.

17. The application according to claim 16, **characterized in that** the biomacromolecule is a polypeptide or a recombinant protein.

18. The application according to claim 17, **characterized in that** the recombinant protein is a genetic engineering antibody, a functional protein with biological activity or a protein formed from the fusion of the Fc fragment.
